# FASCICULE DE BREVET EUROPEEN

(11) **EP 3 806 812 B1**
(45) Date de publication et mention de la délivrance du brevet: **13.04.2022**
(21) Numéro de dépôt: 19742859.2
(22) Date de dépôt: 11.06.2019
(51) Int. Cl.: A61K 8/06, A61K 8/39, A61Q 1/12, A61Q 17/04

(54) **TENSIOACTIF POUR EMULSION EAU DANS HUILE**
TENSID FÜR WASSER-IN-ÖL-EMULSION
SURFACTANT FOR WATER-IN-OIL EMULSION

(30) Priorité: 12.06.2018 FR 1855115
(43) Date de publication de la demande: 21.04.2021
(73) Titulaire: GATTEFOSSE SAS, 69804 Saint-Priest Cedex (FR)
(72) Inventeur: RODIER, Jean-David, 69100 VILLEURBANNE (FR); NOLLET, Maxime, 69003 LYON (FR)
(74) Mandataire: Cabinet Laurent & Charras
(86) Numéro de dépôt international: PCT/FR2019/051411
(87) Numéro de publication internationale: WO 2019/239060

(56) Documents cités:
- FR-A1- 2 936 154
- JP-A- 2008 119 568
- JP-A- 2011 079 753
- JP-A- 2011 193 734
- US-A- 5 840 943
- US-A1- 2015 315 123

## Description

### DOMAINE DE L'INVENTION

La présente invention concerne un tensioactif permettant de formuler des émulsions eau dans huile obtenu à partir d'acides gras et du polyglycérol ayant un degré de polymérisation moyen compris entre 4 et 6.

Le domaine d'utilisation de la présente invention concerne notamment la formulation de compositions cosmétiques.

### ETAT ANTERIEUR DE LA TECHNIQUE

De manière générale, la préparation d'une émulsion de type eau dans huile nécessite l'utilisation d'un agent tensioactif. En effet, en l'absence de tensioactif, l'émulsion n'est généralement pas stable dans le temps et conduit à la formation d'une composition ayant deux phases séparées par une interface.

Le tensioactif permet de stabiliser la dispersion d'une phase aqueuse dans une phase huileuse. Pour cela, il comprend une partie hydrophobe prépondérante par rapport à une partie hydrophile.

A titre d'exemples, des tensioactifs à base de glycérol et d'acide carboxylique gras ont été développés. A cet égard, les acides 12-hydroxystéarique et ricinoléique sont particulièrement intéressants dans la mesure où ils présentent une fonction hydroxyle permettant la polycondensation des acides sur eux-mêmes. Le tensioactif est ensuite obtenu en estérifiant les composés résultant de cette polycondensation sur le glycérol.

La partie hydrophobe du tensioactif correspond aux résidus des acides polycondensés alors que la partie hydrophile correspond au résidu du glycérol.

Ces tensioactifs permettent de formuler divers types de produits cosmétiques comme les fonds de teint contenant des pigments ou les compositions contenant des filtres solaires organiques ou minéraux.

Le document US 5,840,943 décrit un émulsifiant eau dans huile obtenu par estérification de l'acide 12-hydroxystéarique en présence d'un mélange de glycérol, diglycérol, triglycérol, tétraglycérol, pentaglycérol et oligoglycérol.

Le document US 2015/315123 décrit un émulsifiant eau dans huile obtenu par estérification d'un acide carboxylique en C6-C22 en présence d'un polyglycérol ayant un degré de polymérisation moyen compris entre 2 et 16.

Le document WO 2007/027447 décrit également un émulsifiant eau dans huile obtenu par estérification d'un acide carboxylique en présence d'un polyglycérol, par exemple entre l'acide ricinoléique et le polyglycérol-3.

Le polyglycérol étant obtenu par polycondensation du glycérol, il contient généralement du glycérol et un mélange de polyglycérols ayant différents degrés de polymérisation. Ainsi, les émulsifiants obtenus à partir de polyglycérol contiennent un mélange de glycérol estérifié et de polyglycérols estérifiés.

Quand bien même ces émulsifiants permettent de préparer des émulsions eau dans huile stables, il existe toujours un besoin de disposer d'émulsifiants augmentant la stabilité de ces émulsions, que ce soit à la température ambiante ou aux alentours de 40°C.

De manière surprenante, la Demanderesse a résolu ce problème en réduisant ou en éliminant la présence de glycérol lors de la formation d'un émulsifiant à partir de polyglycérol et d'acide(s) carboxylique(s) gras.

### EXPOSE DE L'INVENTION

Le tensioactif selon l'invention est à base de polyglycérol et d'un acide gras de type acide 12-hydroxystéarique (CH₃-(CH₂)₅-CH(OH)-(CH₂)₁₀-C(=O)OH) ou un mélange d'acide 12-hydroxystéarique et d'acide ricinoléique (CH₃-(CH₂)₅-CH(OH)-CH₂-CH=CH-(CH₂)₇-C(=O)OH).

Le polyglycérol utilisé pour préparer ce tensioactif présente un degré de polymérisation (DP) moyen compris entre 4 et 6 c'est-à-dire une macromolécule comprenant, en moyenne, entre quatre et six unités glycérol -(CH₂-CH(OH)-CH₂-O)-.

Le degré de polymérisation correspond au degré de polymérisation moyen en nombre qui est égal au rapport entre la masse molaire moyenne en nombre du polyglycérol et la masse molaire de l'unité glycérol (M = 74g/mol).

Ce tensioactif permet d'obtenir des émulsions plus stables que celles obtenues avec un tensioactif préparé à partir de glycérol ou de polyglycérol présentant un degré de polymérisation moyen inférieur à 4 ou supérieur à 6.

De manière générale, les polyglycérols contiennent une quantité non négligeable de glycérol dont la présence n'est pas un obstacle pour préparer des tensioactifs de type eau dans huile. Toutefois, la Demanderesse a remarqué, que de manière tout à fait inattendue, les tensioactifs préparés en présence d'au moins 3% en poids de glycérol ne permettent pas d'obtenir des émulsions stables dans le temps.

Plus précisément, l'invention concerne un tensioactif eau dans huile constitué d'un mélange de composés de formule (I) : 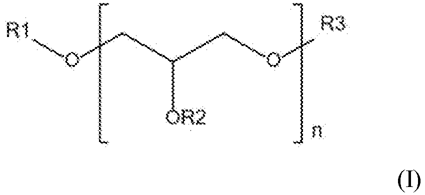
le composé de formule (I) ayant un degré de polymérisation moyen n compris entre 4 et 6, R1, R2 et R3 étant, indépendamment l'un de l'autre, un atome d'hydrogène ou un groupement R4 dérivé d'au moins un acide carboxylique,
l'acide carboxylique étant l'acide 12-hydroxystéarique ou un mélange d'acide 12-hydroxystéarique et d'acide ricinoléique,
R4 étant un groupement C(=O)-(R5)-CH(OH)-(CH₂)₅-CH₃ ou C(=O)-(R5)-CH[(CH₂)₅-CH_{3]}-O-{C(=0)-(R5)-CH[(CH₂)₅-CH_{3]}-O}ₚC(=O)-(R5)-CH[(CH₂)₅-CH_{3]}-OH avec p = 0 à 5, avantageusement 0 à 3, plus avantageusement 0 à 2,
R5 étant un groupement (CH₂)₁₀ ou un mélange de groupements (CH₂)₁₀ et (CH₂)₇-(CH=CH)-CH₂,
au moins un des groupes R1, R2 et R3 étant un groupement R4.

p est avantageusement un nombre entier de 0 à 5, plus avantageusement de 0 à 3, et encore plus avantageusement de 0 à 2.

La structure de R4 peut être présentée comme suit : 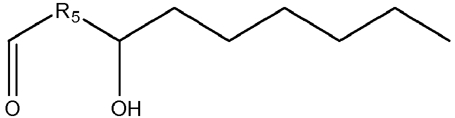 ou

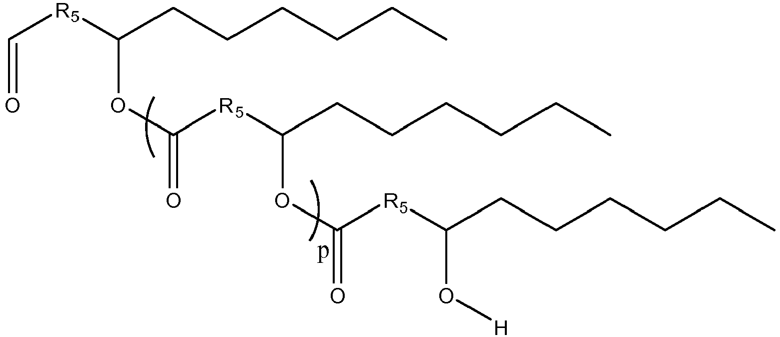

Le tensioactif eau dans huile selon l'invention est le résultat de la réaction d'estérification entre le polyglycérol et un ou plusieurs acides carboxyliques, aptes à être également estérifiés par polycondensation des acides sur eux-mêmes. Ces deux types d'estérification sont réalisés simultanément.

Le composé de formule (I) a un degré de polymérisation moyen n compris entre 4 et 6, avantageusement entre 4 et 5, plus avantageusement 4,5 à 5. Le degré de polymérisation moyen en nombre est déterminé par chromatographie en phase gazeuse couplée à un détecteur à ionisation de flamme.

Le tensioactif selon l'invention présente une valeur HLB avantageusement comprise entre 1 et 6, plus avantageusement entre 2 et 6, et encore plus avantageusement entre 3 et 5.

De manière générale, la valeur HLB correspond à la balance hydrophile-lipophile du tensioactif. Elle varie de 0 à 20, les valeurs inférieures à 6 correspondent à des tensioactifs permettant de formuler des émulsions eau dans huile, les valeurs supérieures à 8 correspondent à des tensioactifs permettant de formuler des émulsions huile dans eau. Elle est déterminée selon la méthode de Griffin (Griffin WC, Classification of Surface-Active Agents by HLB, Journal of the Society of Cosmetic Chemists, 1949, 1, pages 311-326).

Le composé de formule (I) comprend au moins un groupement R4 qui est un dérivé de l'acide 12-hydroxystéarique ou d'un mélange d'acide 12-hydroxystéarique et d'acide ricinoléique. Il peut comprendre plusieurs groupements R4, identiques ou différents les uns des autres. Avantageusement, les groupements R1 ct R3 sont des groupements R4.

Lorsque R4 est un groupement C(=O)-(R5)-CH[(CH₂)₅-CH_{3]}-O-{C(=O)-(R5)-CH[(CH₂)₅-CH_{3]}-O}ₚC(=O)-(R5)-CH[(CH₂)₅-CH_{3]}-OH, p est un nombre entier avantageusement compris entre 0 et 5, plus avantageusement entre 0 et 3.

Le composé de formule (I) ayant un degré de polymérisation moyen compris entre 4 et 6, il comprend en moyenne entre 4 et 6 groupements R2, avantageusement entre 4 et 5, plus avantageusement 4,5 à 5. Ces groupements R2 peuvent être un atome d'hydrogène et/ou un groupement R4. Il s'agit avantageusement d'un atome d'hydrogène pour tous les groupements R2. Dans ce cas, seuls les OH terminaux du polyglycérol peuvent être avantageusement estérifiés et correspondre à R4.

Dans la formule (I), le rapport molaire entre les groupements R4 (en position R1 et/ou R2 et/ou R3) et les unités (-CH₂-CH(O-)-CH₂-O-) du polyglycérol est avantageusement compris entre 1 et 6, plus avantageusement entre 2 et 4.

L'acide 12-hydroxystéarique et l'acide ricinoléique présentent la particularité de comprendre, tous les deux, une fonction hydroxyle OH et une fonction acide carboxylique COOH. Ces deux types de fonctions peuvent réagir entre elles par estérification (polycondensation) entrainant la formation d'une macromolécule d'acide carboxylique ou d'un mélange d'acides carboxyliques.

Comme déjà indiqué, cette réaction de polycondensation est réalisée simultanément à la réaction d'estérification du polyglycérol. Dans ce cas, le groupement R4 est avantageusement un mélange de groupements dans lesquels R5 = (CH₂)₁₀ et/ou (CH₂)₇-(CH=CH)-CH₂.

Ainsi, lorsque le tensioactif selon l'invention est préparé à partir d'un mélange d'acide 12-hydroxystéarique et d'acide ricinoléique, le groupement R4 peut comprendre des groupements R5 de type (CH₂)₁₀ (acide 12-hydroxystéarique) mais aussi des groupements R5 de type (CH₂)₇-(CH=CH)-CH₂ (acide ricinoléique).

Le tensioactif peut se présenter sous la forme d'un copolymère bloc de type ABA. C'est notamment le cas lorsque R2 est un atome d'hydrogène et lorsque R1 et R3 sont tous les deux un poly(acide 12-hydroxystéarique) ou un poly(acide ricinoléique).

Lorsqu'un mélange d'acides carboxyliques est mis en œuvre, le rapport entre le nombre de groupements R5 de type (CH₂)₁₀ (acide 12-hydroxystéarique) et le nombre groupements R5 de type (CH₂)₇-(CH=CH)-CH₂ (acide ricinoléique) est avantageusement compris entre 0,5 et 4, plus avantageusement entre 0,75 et 2, encore plus avantageusement entre 1 et 2.

Le tensioactif selon l'invention peut être sous forme solide ou liquide visqueux. Cependant, la présence simultanée de résidus d'acide 12-hydroxystéarique et de résidus d'acide ricinoléique permet de modifier la nature du tensioactif, par exemple, en rendant ce dernier visqueux ou fluide.

Selon un mode de réalisation particulier, R5 = (CH₂)₁₀. En d'autres termes, R4 est un dérivé de l'acide 12-hydroxystéarique, et plus particulièrement un groupement C(=O)-(CH₂)₁₀-CH(OH)-(CH₂)₅-CH₃ ou un poly(acide 12-hydroxystéarique) de formule C(=O)-(CH₂)₁₀-CH[(CH₂)₅-CH_{3]}-O-{C(=O)-(CH₂)₁₀-CH[(CH₂)₅-CH_{3]}-0}ₚC(=O)-(CH₂)₁₀-CH[(CH₂)₅-CH_{3]}-OH.

Selon un autre mode de réalisation particulier, R5 peut être un mélange de groupements (CH₂)₁₀ et (CH₂)₇-(CH=CH)-CH₂. En d'autres termes, R4 est un dérivé de l'acide ricinoléique et de l'acide 12-hydroxystéarique, et plus particulièrement un mélange de groupements C(=O)-(CH₂)₇-(CH=CH)-CH₂-CH(OH)-(CH₂)_{S}-CH₃ et C(=O)-(CH₂)₁₀-CH[(CH₂)₅-CH₃]-O-{C(=O)-(CH₂)₁₀-CH[(CH₂)₅-CH_{3]}-O}ₚC(=O)-(CH₂)₁₀-CH[(CH₂)₅-CH₃]-OH ou un poly(acide ricinoléique - acide 12-hydroxystéarique).

Selon un autre mode de réalisation particulier, R2 est un atome d'hydrogène, et R1 et R3 sont, indépendamment l'un de l'autre, un poly(acide 12-hydroxystéarique), un poly(acide ricinoléique) ou un poly(acide 12-hydroxystéarique - acide ricinoléique).

Ainsi, R1 et R3 peuvent être des groupements R4 de formule C(=O)-(CH₂)₁₀-CH[(CH₂)₅-CH₃]-O-{C(=O)-(CH₂)₁₀-CH[(CH₂)₅-CH₃]-O}ₚC(=O)-(CH₂)₁₀-CH[(CH₂)₅-CH₃]-OH et/ou de formule C(=O)-(CH₂)₇-(CH=CH)-CH₂-CH[(CH₂)₅-CH₃]-O- {C(=O)-(CH₂)₇-(CH=CH)-CH₂-CH[(CH₂)₅-CH₃]-O}ₚC(=O)-(CH₂)₇-(CH=CH)-CH₂-CH[(CH₂)₅-CH₃]-OH.

L'utilisation d'un mélange d'acides carboxyliques permet de moduler l'aspect du tensioactif, par exemple en le rendant plus visqueux, voire liquide à la température ambiante (20°C). Ainsi, ce mode de réalisation peut s'avérer particulièrement attrayant pour préparer des émulsions eau dans huile à des températures avantageusement comprises entre 10 et 40°C, plus avantageusement entre 15 et 30°C.

La présente invention concerne également un procédé de préparation de ce tensioactif, par réaction d'estérification entre le polyglycérol et au moins un acide carboxylique. L'acide carboxylique est l'acide 12-hydroxystéarique ou un mélange d'acide 12-hydroxystéarique et d'acide ricinoléique.

Le polyglycérol présente un degré de polymérisation moyen en nombre compris entre 4 et 6. Il présente une masse molaire moyenne en nombre avantageusement comprise entre 314 g/mol et 462 g/mol. En outre, il comprend avantageusement moins de 3% en masse de glycérol (HO-CH₂-CH(OH)-CH₂-OH), plus avantageusement moins de 2% en masse, et encore plus avantageusement moins de 1% en masse, en poids par rapport au poids du polyglycérol (DP n = 4 à 6).

Le polyglycérol est préférentiellement dépourvu de glycérol.

La réaction d'estérification est avantageusement réalisée en présence d'un rapport molaire polyglycérol / acide(s) carboxylique(s) compris entre 1 et 0,15, plus avantageusement entre 0,5 et 0,25.

Lorsqu'un mélange d'acides carboxyliques est utilisé, le rapport molaire acide 12-hydroxystéarique / acide ricinoléique est avantageusement compris entre 0,5 et 4, plus avantageusement entre 0,75 et 2.

La réaction d'estérification est avantageusement réalisée à une température comprise entre 185 et 235°C, plus avantageusement entre 190 et 220°C.

De manière générale, l'eau générée lors de la formation du tensioactif peut être éliminée, de manière continue ou discontinue, au cours de la réaction, par exemple sous un courant d'azote. Cela permet d'éviter le phénomène de rétro-estérification.

D'autre part, le procédé peut être réalisé en présence d'un catalyseur, par exemple des ions hydroxyde pour l'estérification du polyglycérol.

Le procédé de préparation du tensioactif selon l'invention est avantageusement réalisé en l'absence de solvant.

Comme déjà indiqué, la réaction de polycondensation du ou des acides carboxyliques est réalisée en même temps que la réaction d'estérification du polyglycérol.

La présente invention concerne également une composition tensioactive comprenant le tensioactif selon l'invention. Cette composition est réalisée à partir de polyglycérol ayant un degré de polymérisation moyen compris entre 4 et 6. Elle est avantageusement réalisée en présence de moins de 3% de glycérol (HO-CH₂-CH(OH)-CH₂-OH), avantageusement moins de 2%, plus avantageusement moins de 1%, en poids par rapport au poids du polyglycérol (DP n = 4 à 6).

Comme déjà indiqué, la diminution de la quantité de glycérol permet d'augmenter la stabilité des émulsions préparées à partir du tensioactif ou de la composition tensioactive selon l'invention.

Le tensioactif et la composition tensioactive selon l'invention peuvent être utilisés pour formuler des émulsions eau dans huile, notamment dans le domaine de la cosmétique.

En outre, la présente invention concerne également une composition cosmétique comprenant ce tensioactif ou cette composition tensioactive.

L'invention et les avantages qui en découlent ressortiront mieux des exemples suivants donnés afin d'illustrer l'invention et non de manière limitative.

### EXEMPLES DE REALISATION DE L'INVENTION

De manière générale, des analyses en GC-FID (chromatographie en phase gazeuse couplée à un détecteur à ionisation de flamme) ont été réalisées pour déterminer la composition de polyglycérols utilisés et donc leur degré de polymérisation, également appelé DPn.

### Exemple 1 : Tensioactif selon l'invention préparé à partir de l'acide 12-hydroxystéarique et du polyglycérol (DPn = 4, 6)

Chargement de 378,6 g d'acide 12-hydroxystéarique et de 121,4 g de polyglycérol dans un réacteur. Le milieu réactionnel est chauffé à 210°C sous azote. L'eau résultant de la réaction d'estérification est soutirée sous un courant d'azote pour déplacer l'équilibre. Après 6 heures de réaction, le réacteur est vidangé et le produit de réaction (tensioactif) est filtré sur membrane de polypropylène.

Ce produit a été mis en œuvre dans une émulsion eau dans huile de la façon décrite dans le tableau 1. Il est sous forme solide à 20°C.

**Tableau 1 : Composition de la formulation préparée à partir de l'exemple 1.**

| **Ingrédients** | **Pourcentage massique** |
|---|---|
| Tensioactif selon l'exemple 1 | 5 |
| Dicaprylyl carbonate | 25 |
| Conservateur :phénoxyéthanol et éthylhexylglycérine (90%/10% en masse) | 1 |
| Eau | 68 |
| NaCl | 0,5 |
| MgSO₄ | 0,5 |

Le protocole de préparation de cette émulsion est le suivant :
Le tensioactif (exemple 1) est solubilisé dans la phase huileuse (dicaprylyl carbonate) et les deux sels (NaCl, MgSO₄) sont solubilisés dans l'eau. Les deux phases sont ensuite chauffées au bain marie jusqu'à 75°C. Une fois la température atteinte, la phase huileuse est agitée à l'aide d'un homogénéiseur à 2000 tour/min. La phase aqueuse est ensuite ajoutée lentement dans la phase huileuse. Une fois cette addition finie, l'homogénéisation à 2000 tour/min est maintenue pendant 20 minutes. Ensuite, l'émulsion est placée dans un bain marie d'eau froide (20°C) pour diminuer la température de l'émulsion à 30°C. Au moment de la mise en place du bain marie froid, le conservateur est ajouté au goutte à goutte et l'agitation est diminuée à 1500 tour/min. L'agitation est ensuite maintenue pendant 10 minutes. L'émulsion est ensuite placée dans un contenant pour le suivi en stabilité.

La stabilité de cette émulsion a été suivie à température ambiante, à 40°C et après deux cycles de gel / dégel de la formulation (tableau 2).

**Tableau 2 : Stabilité de la formulation préparée à partir de l'exemple 1.**

| **Conditions** | **Observations** |
|---|---|
| Température ambiante | Stable pendant au moins 3 mois |
| 40°C | Stable pendant au moins 1 mois |
| Cycle gel/dégel | Stable |

### Exemples 2 à 4 : Tensioactifs préparés à partir d'un mélange d'acides carboxyliques et de polyglycérol (DPn = 4,6)

L'utilisation d'un mélange de l'acide 12-hydroxystéarique et de l'acide ricinoléique permet d'obtenir un produit visqueux plus facile à manipuler que le tensioactif selon l'exemple 1. Le tensioactif ainsi obtenu permet de réaliser des formulations à froid (entre 20 et 25°C).

Exemple 2 selon l'invention : Chargement de 171,2 g d'acide ricinoléique, 172,6 g d'acide 12-hydroxystéarique et de 121,4 g de polyglycérol dans un réacteur. Le milieu réactionnel est chauffé à 210°C sous azote. L'eau résultant de la réaction d'estérification est soutirée sous un courant d'azote pour déplacer l'équilibre. Après 6 heures de réaction, le réacteur est vidangé et le produit de réaction (tensioactif) est filtré sur membrane de polypropylène.

Les exemples comparatifs 3 et 4 ont été réalisés selon le protocole de l'exemple 2 mais avec les modifications suivantes :
Exemple 3 : 5% de la masse du polyglycérol sont remplacés par du glycérol.
Exemple 4 : 30% de la masse du polyglycérol sont remplacés par du glycérol.

**Tableau 3 : Composition des émulsions préparées à partir des exemples 2 à 4.**

| **Ingrédients** | **Pourcentage massique** |
|---|---|
| Tensioactif selon l'exemple 2, 3 ou 4 | 5 |
| Triglycérides à chaines moyennes | 25 |
| Conservateur :phénoxyéthanol et éthylhexylglycérine (90%/10% en masse) | 1 |
| Eau | 67 |
| NaCl | 1 |
| MgSO₄ | 1 |

Le protocole de préparation de ces émulsions est identique à celui de l'exemple 1.

La stabilité des émulsions obtenues à partir des exemples 2,3 et 4 a été suivie à l'aide d'un appareil de type Turbiscan^{™} (tableau 3). Celui-ci mesure le pourcentage de rétrodiffusion moyen de chaque composition. Plus le pourcentage est élevé, plus l'émulsion est opaque et donc fine. Lorsque ce pourcentage évolue, cela traduit une évolution de la formulation et donc de sa stabilité (tableau 4).

**Tableau 4 : Stabilité des émulsions préparées à partir des exemples 2 à 4.**

| **Tensioactif** | **exemple 2** | **exemple 3** | **exemple 4** |
|---|---|---|---|
| Pourcentage massique de glycérol ajouté dans le polyglycérol | 0 | 5 | 30 |

| **Rétrodiffusion moyenne (%)** | | | |
|---|---|---|---|
| T0 | 86,1 | 83,3 | 68,8 |
| 24 heures à 40°C | 84,2 | 63,2 | 47,5 |
| 1 semaine 40°C | 78,3 | 40,7 | 48,2 |

| **Rétrodiffusion moyenne normalisée (%)** | | | |
|---|---|---|---|
| T0 | 100,0 | 100,0 | 100,0 |
| 24 heures à 40°C | 97,8 | 75,9 | 69,0 |
| une semaine à 40°C | 90,9 | 48,9 | 70,1 |

L'émulsion obtenue à partir du tensioactif selon l'exemple comparatif 4 est moins opaque à T0 que les autres compositions. L'émulsion est moins fine.

En normalisant les rétrodiffusions moyennes, on remarque que les formules faites avec les tensioactifs des exemples comparatifs 3 et 4 ont une chute de la rétrodiffusion moyenne plus importante que celles obtenue à partir de l'exemple 2 selon l'invention.

En conclusion, les tensioactifs des exemples comparatifs 3 et 4 stabilisent moins bien la formule que le tensioactif selon l'exemple 2 selon l'invention.

En d'autres termes, les tensioactifs préparés à partir de polyglycérol exempt de glycérol donnent des émulsions plus stables que les tensioactifs préparés à partir de polyglycérol comprenant 5% ou 30% de glycérol.

### Exemples 5 et 6 : Tensioactifs préparés à partir de polyglycérol 3,1 ou de polyglycérol 4,6

L'effet de la composition du polyglycérol sur les performances de leurs esters de polyhydroxystearate en formulation a été analysé.

Tout d'abord, des analyses en GC-FID (chromatographie en phase gazeuse couplée à un détecteur à ionisation de flamme) ont été réalisées pour déterminer la composition de deux polyglycérols de degré de polymérisation différent. Les résultats sont reportés dans le tableau 5.

**Tableau 5 : Composition des polyglycérol A et B obtenue par chromatographie en phase gazeuse**

| | Composition (%) | |
|---|---|---|
| | polyglycérol A | polyglycérol B |
| glycérol | 2,17 | 0,14 |
| diglycérol | 23,68 | 9,47 |
| polyglycérol-3 | 48,35 | 27,44 |
| polvglycérol-4 | 18,28 | 21,71 |
| polyglycérol-5 | 5,63 | 14,46 |
| polyglycérol-6 | 1,56 | 9,81 |
| polyglycérol-7 | 0,32 | 6,82 |
| polyglycérol-7 | 0,02 | 4,59 |
| polyglycérol-9 | 0 | 2,87 |
| polyglycérol-10 à polyglycérol-13 | 0 | 2,72 |
| | | |
| **Mn** (g/mol) | 245,63 | 359,12 |
| **DPn** (nombre de motif glycérol) | 3,1 | 4,6 |

Le polyglycérol B a également été utilisé pour réaliser les exemples 1 à 4.

Ensuite, deux tensioactifs ont été synthétisés à partir des polyglycérols A et B selon le protocole suivant :
Exemple 5 : Chargement de 414,5 g d'acide 12-hydroxystéarique et de 85 g de polyglycérol A dans un réacteur. Le milieu réactionnel est chauffé à 215°C sous azote. Après 6 heures de réaction, le réacteur est vidangé et l'ester filtré sur membrane de polypropylène.
Exemple 6 selon l'invention : Chargement de 359 g d'acide 12-hydroxystéarique et de 140,5 g de polyglycérol B dans un réacteur. Le milieu réactionnel est chauffé à 215°C sous azote. Après 5 heures de réaction, le réacteur est vidangé et l'ester filtré sur membrane de polypropylène.

Il est à noter que les masses d'acide gras et polyglycérol dans les exemples 5 et 6 ont été choisies pour garder le rapport molaire équivalent entre l'acide et le polyglycérol dans les deux exemples.

Les deux tensioactifs ainsi obtenus ont été mis en œuvre dans une formule eau dans huile (tableau 6).

**Tableau 6 : Composition des émulsions préparées à partir des tensioactifs des exemples 5 et 6.**

| Ingrédients | Pourcentage massique |
|---|---|
| Tensioactif de l'exemple 5 ou 6 | 5 |
| Dicaprylyl carbonate | 25 |
| Conservateur :phénoxyéthanol et éthylhexylglycérine (90%/10% en masse) | 1 |
| Eau | 68 |
| NaCl | 0,5 |
| MgSO₄ | 0,5 |

Le protocole de préparation des formules est le suivant :
Le tensioactif est solubilisé dans la phase huileuse et les deux sels sont solubilisés dans l'eau. Les deux phases sont ensuite chauffées au bain marie jusqu'à 75°C. Une fois la température atteinte, la phase huileuse est agitée à l'aide d'un homogénéiseur à 2000 tr/min (tours par minute). La phase aqueuse est ensuite ajoutée lentement dans la phase huileuse. Une fois l'ajout d'eau fini, l'homogénéisation à 2000 tr/min est maintenue pendant 20 minutes. Ensuite l'émulsion est placée dans un bain marie d'eau froide (20°C) pour diminuer la température de l'émulsion à 30°C. Au moment de la mise en place du bain-marie froid, le conservateur est ajouté au goutte à goutte et l'agitation est diminuée à 1500 tr/min. L'agitation est ensuite maintenue pendant 10 minutes. L'émulsion est ensuite placée dans un contenant pour le suivi en stabilité.

La stabilité des deux formulations a été suivie à température ambiante, à 40°C et après deux cycles de gel / dégel de la formulation.

Les résultats sont répertoriés dans le tableau 7.

**Tableau 7 : Stabilité des formulations préparées à partir des tensioactifs des exemples 5 et 6.**

| Tensioactif | Exemple 5 | Exemple 6 |
|---|---|---|
| Température ambiante | Stable moins d'un mois | Stable plus de 3 mois |
| 40°C | Stable moins de 3 jours | Stable plus de 7 jours |
| Cycle gel/dégel | Stable | Stable |

La stabilité des formulations utilisant le tensioactif B (issu du polyglycérol B) selon l'invention est supérieure quelles que soient les conditions d'observations. En revanche, la formulation comprenant le tensioactif A (issu du polyglycérol A) est nettement moins stable.

La composition du polyglycérol initial a donc un impact important sur la performance du tensioactif. La présence de glycérol ou de polyglycérol à bas degré de polymérisation (inférieur à 4) induit une contre-performance du tensioactif. Il est donc préférable d'utiliser un polyglycérol de degré de polymérisation DP moyen en nombre compris entre 4 et 6.

### Exemple 7: Tensioactifs préparés à partir de polyglycérol 3,1 ou de polyglycérol 10 (DPn = 10)

Exemple 7 : Chargement de 323,26 g d'acide 12-hydroxystéarique et de 151,7 g de polyglycérol C de DPn = 10 dans un réacteur. Le catalyseur est ajouté sous agitation. Le milieu réactionnel est chauffé sous azote. Lorsque l'indice d'acide est inférieur à 2mgKOH/g le chauffage est arrêté. Le réacteur est vidangé et l'ester filtré sur membrane de polypropylène.

Ce produit a été mis en œuvre dans une formule eau dans huile (tableau 8).

**Tableau 8 : Composition de l'émulsion préparée à partir du tensioactif de l'exemple 7.**

| ingrédients | Pourcentage massique |
|---|---|
| Tensioactif de l'exemple 7 | 5 |
| Dicaprylyl carbonate | 25 |
| Conservateur : phénoxyéthanol et éthylhexylglycérine (90%/10% en masse) | 1 |
| Eau | 68 |
| NaCl | 0,5 |
| MgSO₄ | 0,5 |

Le protocole de préparation de cette formule est identique à celui décrit pour les exemples 5 et 6.

Les résultats sont répertoriés dans le tableau 9.

**Tableau 9 : Stabilité de la formulation préparée à partir du tensioactif de l'exemple 7.**

| Tensioactif | Exemple 7 |
|---|---|
| Température ambiante | Stable moins d'un mois |
| 40°C | Stable moins d'un de 7 jours |
| Cycle gel/dégel | instable |

La stabilité de la formule utilisant le tensioactif de l'exemple 7 est inférieure, quelles que soient les conditions d'observations, à celle des tensioactifs des exemples 5 et 6 (voir le tableau 7).

En résumé, la composition du polyglycérol a un impact important sur la performance des formulations. La présence initiale de petit ou grand polyglycérol induit une contre-performance du tensioactif. Il est donc préférable d'utiliser un polyglycérol de degré de polymérisation DPn entre 4 et 6 soit une masse molaire entre 314 g/mol et 462 g/mol.

### Effet du degré de polymérisation du polyglycérol ou de la composition de l'acide gras hydroxvlé sur les performances de leurs esters en formulation

Deux tensioactifs ont été comparés :
- le premier (exemple 2) est issu de l'estérification entre un polyglycérol (DPn = 4,6) l'acide ricinoléique et l'acide 12-hydroxystéarique,
- le second (exemple 8) est issu de l'estérification entre un polyglycérol (DPn = 10) et un mélange d'acide 12-hydroxystéarique et d'acide ricinoléique.

### Synthèse

Exemple 8 : Chargement de 132 g d'acide 12-hydroxystéarique, 131 g d'acide ricinoléique et de 237 g de polyglycérol de DPn=10 dans un réacteur. 0,085 g de soude est ajouté sous agitation. Le milieu réactionnel est chauffé à 215°C sous azote. Après 6 heures de réaction, l'indice d'acide mesuré est 1,4 mg KOH/g. Le réacteur est vidangé et l'ester filtré sur membrane de polypropylène.

Il est à noter que les masses d'acides et de polyglycérol de l'exemple 8 ont été choisies de manière à avoir un rapport molaire entre les acides et le polyglycérol identique au rapport molaire de l'exemple 2.

### Performances en formulations

Ces deux produits (exemples 2 et 8) ont été mis en œuvre dans une formule eau dans huile contenant des pigments de la façon décrite ci-dessous :

| Phase | Ingrédients | Pourcentage massique |
|---|---|---|
| Huileuse | Tensioactif de l'exemple 2 ou 8 | 5 |
| | Triglycérides à chaîne moyenne (TCM) | 3.25 |
| | Lanol 99 | 9.5 |
| | Eutanol G | 9.43 |
| | Conservateur | 0.7 |
| | Pigment jaune | 9.3 |
| | Pigment blanc | 1.12 |
| | Pigment rouge | 0.5 |
| Aqueuse | Eau | 54.2 |
| | Glycérine | 5 |
| | NaCl | 1 |
| | MgSO₄ | 1 |

### Le protocole de préparation des formules est le suivant :

Le tensioactif est solubilisé dans la phase huileuse et les deux sels (NaCl et MgSO₄) sont solubilisés dans l'eau. Les deux phases sont ensuite chauffées au bain marie jusqu'à 75°C. Une fois la température atteinte, la phase huileuse est agitée à l'aide d'un homogénéiseur à 2000 tr/min. La phase aqueuse est ensuite ajoutée lentement dans la phase huileuse. Une fois l'ajout d'eau fini, l'homogénéisation à 2000 tr/min est maintenue pendant 20 minutes. Ensuite, l'émulsion est placée dans un bain marie d'eau froide pour diminuer la température de l'émulsion à 30°C. Au moment de la mise en place du bain-marie froid, le conservateur est ajouté au goutte à goutte et l'agitation est diminuée à 1500 tr/min. L'agitation est ensuite maintenue pendant 10 minutes. L'émulsion est ensuite placée dans un contenant pour le suivi en stabilité.

La stabilité des deux formulations a été suivie à 40°C.

Les résultats sont les suivants :

| Essai avec l'exemple | 2 | 8 |
|---|---|---|
| 40°C | Stable plus de 14 jours | Stable moins de 14 jours |

Une sédimentation de la formule contenant le tensioactif selon l'exemple 8 est à noter. La stabilité de la formule utilisant l'exemple 8 est donc inférieure à la formule utilisant le tensioactif selon l'exemple 2.

Le degré de polymérisation (DPn) du polyglycérol a un impact important sur la performance des formulations. L'utilisation d'un polyglycérol de DPn supérieur à 6 induit une contre-performance du tensioactif. Il est donc préférable d'utiliser un polyglycérol de degré de polymérisation DPn entre 4 et 6 soit une masse molaire entre 314 g/mol et 462 g/mol.

### Effet du degré de la composition en acide gras sur les performances en formulation

Deux tensioactifs ont été comparés :
- le premier (exemple 2) est issu de l'estérification entre un polyglycérol (DPn = 4,6) l'acide ricinoléique et l'acide 12-hydroxystéarique,
- le second (exemple 9) est issu de l'estérification entre un polyglycérol (DPn = 4,6) et l'acide ricinoléique.

Exemple 9 : Chargement de 369,5 g d'acide ricinoléique et de 130,5 g de polyglycérol de DPn=4,6 dans un réacteur. 0,085 g de soude est ajouté sous agitation. Le milieu réactionnel est chauffé à 215°C sous azote. Après 6 heures de réaction, l'indice d'acide mesuré est 1,0 mg KOH/g. Le réacteur est vidangé et l'ester filtré sur membrane de polypropylène.

Il est à noter que les masses d'acide et de polyglycérol de l'exemple 9 ont été choisies de manière à avoir un rapport molaire entre l'acide et le polyglycérol identique au rapport molaire de l'exemple 2.

### Performances en formulations

Ces deux produits ont été mis en œuvre dans une formule eau dans huile contenant des pigments de la façon décrite ci-dessous :

| Phase | Ingrédients | Pourcentage massique |
|---|---|---|
| Huileuse | Tensioactif de l'exemple 2 ou 9 | 5 |
| | Triglycérides à chaîne moyenne (TCM) | 3.25 |
| | Lanol 99 | 9.5 |
| | Eutanol G | 9.43 |
| | Conservateur | 0.7 |
| | Pigment jaune | 9.3 |
| | Pigment blanc | 1.12 |
| | Pigment rouge | 0.5 |
| Aqueuse | Eau | 54.2 |
| | Glycérine | 5 |
| | NaCl | 1 |
| | MgSO₄ | 1 |

Le protocole de préparation des formules est le suivant :
Le tensioactif est solubilisé dans la phase huileuse et les deux sels (NaCl et MgSO₄) sont solubilisés dans l'eau. Les deux phases sont ensuite chauffées au bain marie jusqu'à 75°C. Une fois la température atteinte, la phase huileuse est agitée à l'aide d'un homogénéiscur à 2000 tr/min. La phase aqueuse est ensuite ajoutée lentement dans la phase huileuse. Une fois l'ajout d'eau fini, l'homogénéisation à 2000 tr/min est maintenue pendant 20 minutes. Ensuite l'émulsion est placée dans un bain marie d'eau froide pour diminuer la température de l'émulsion à 30°C. Au moment de la mise en place du bain-marie froid, le conservateur est ajouté au goutte à goutte et l'agitation est diminuée à 1500 tr/min. L'agitation est ensuite maintenue pendant 10 minutes. L'émulsion est ensuite placée dans un contenant pour le suivi en stabilité.

La stabilité des deux formulations a été suivie à 40°C.

Les résultats sont les suivants :

| | | |
|---|---|---|
| Essai avec l'exemple | 2 | 9 |
| 40°C | Stable plus de 14 jours | Stable moins de 7 jours |

Un déphase rapide de la formule contenant le tensioactif selon l'exemple 9 est à noter. La stabilité de la formule utilisant l'exemple 9 est donc inférieure à la formule utilisant le tensioactif selon l'exemple 2.

En résumé, l'utilisation unique de l'acide ricinoléique, au lieu de l'acide 12-hydroxystéarique ou d'un mélange d'un mélange d'acides 12-hydroxystéarique et ricinoléique, lors de la synthèse du tensioactif, induit une performance plus faible. Il est donc préférable d'utiliser l'acide 12-hydroxystéarique ou un mélange des deux acides ricinoléique et 12-hydroxystéarique au lieu de l'utilisation de l'acide ricinoléique seul.

## Revendications

1. Tensioactif eau dans huile constitué d'un mélange de composés de formule (I) : 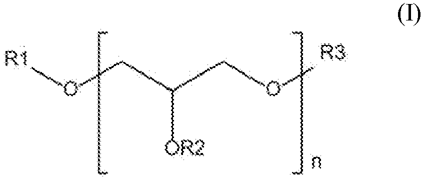
le composé de formule (I) ayant un degré de polymérisation moyen n compris entre 4 et 6,
R1, R2 et R3 étant, indépendamment l'un de l'autre, un atome d'hydrogène ou un groupement R4 dérivé d'au moins un acide carboxylique,
l'acide carboxylique étant l'acide 12-hydroxystéarique ou un mélange d'acide 12-hydroxystéarique et d'acide ricinoléique,
R4 étant un groupement C(=O)-(R5)-CH(OH)-(CH₂)₅-CH₃ ou C(=O)-(R5)-CH[(CH₂)₅-CH_{3]}-o-{C(=o)-(R5)-CH[(CH₂)₅-CH_{3]}-o}ₚC(=o)-(R5)-CH[(CH₂)₅-CH₃]-OH avec p = 0 à 5,
R5 étant un groupement (CH₂)₁₀ ou un mélange de groupements (CH₂)₁₀ et (CH₂)₇-(CH=CH)-CH₂,
au moins un des groupes R1, R2 et R3 étant un groupement R4.

2. Tensioactif selon la revendication 1, ***caractérisé* en ce que** le composé de formule (I) a un degré de polymérisation moyen n compris entre 4 et 5, avantageusement 4,5 à 5.

3. Tensioactif selon la revendication 1 ou 2, ***caractérisé* en ce que** le tensioactif présente une valeur HLB comprise entre 1 et 6, avantageusement entre 2 et 6, plus avantageusement entre 3 et 5.

4. Tensioactif selon l'une des revendications 1 à 3, ***caractérisé* en ce que** p = 0 à 3, avantageusement p = 0 à 2.

5. Tensioactif selon l'une des revendications 1 à 4, ***caractérisé* en ce que** R2 est un atome d'hydrogène.

6. Tensioactif selon l'une des revendications 1 à 5, ***caractérisé* en ce qu'**il présente un rapport entre le nombre de groupements R5 = (CH₂)₁₀ et le nombre groupements R5 = (CH₂)₇-(CH=CH)-CH₂ compris entre 0,5 et 4, avantageusement entre 0,75 et 2.

7. Tensioactif selon l'une des revendications 1 à 6, ***caractérisé* en ce que** R1 et R3 sont des groupements R4
de formule C(=O)-(CH₂)₁₀-CH[(CH₂)₅-CH_{3]}-O- {C(=O0)-(CH₂)₁₀-CH[(CH₂)₅-CH_{3]}-O}ₚC(=O)-(CH₂)₁₀-CH[(CH₂)₅-CH₃]-OH
ou
un mélange de groupements de formule C(=O)-(CH₂)₁₀-CH[(CH₂)₅-CH₃]-O-{C(=O)-(CH₂)₁₀-CH[(CH₂)₅-CH₃]-O}ₚC(=O)-(CH₂)₁₀-CH[(CH₂)₅-CH₃]-OH et de formule C(=O)-(CH₂)₇-(CH=CH)-CH₂-CH[(CH₂)₅-CH₃]-O- {C(=O)-(CH₂)₇-(CH=CH)-CH₂-CH[(CH₂)₅-CH_{3]}-O}ₚC(=O)-(CH₂)₇-(CH=CH)-CH₂-CH[(CH₂)₅-CH_{3]}-OH.

8. Procédé de préparation du tensioactif selon l'une des revendications 1 à 7 par réaction d'estérification entre le polyglycérol et au moins un acide carboxylique, l'acide carboxylique étant l'acide 12-hydroxystéarique ou un mélange d'acide 12-hydroxystéarique et d'acide ricinoléique.

9. Procédé selon la revendication 8, ***caractérisé* en ce que** la réaction d'estérification est réalisée en présence d'un rapport molaire polyglycérol / acide(s) carboxylique(s) compris entre 1 et 0,15, plus avantageusement entre 0,5 et 0,25.

10. Procédé selon la revendication 8 ou 9, ***caractérisé* en ce que** la réaction d'estérification est réalisée à une température comprise entre 185 et 235°C, avantageusement entre 190 et 220°C.

11. Composition tensioactive comprenant le tensioactif selon l'une des revendications 1 à 7.

12. Composition cosmétique comprenant le tensioactif selon l'une des revendications 1 à 7 ou la composition tensioactive selon la revendication 11.

## Patentansprüche

1. Wasser-in-Öl-Tensid bestehend aus einer Mischung von Verbindungen der Formel (I): 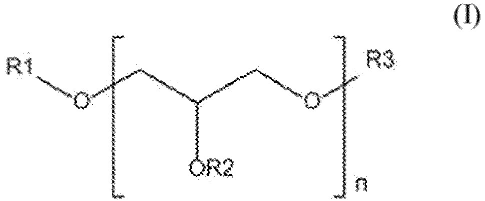
wobei die Verbindung der Formel (I) einen durchschnittlichen Polymerisationsgrad n zwischen 4 und 6 aufweist,
R1, R2 und R3 unabhängig voneinander ein Wasserstoffatom oder eine von mindestens einer Carbonsäure abgeleitete Gruppe R4 sind,
wobei die Carbonsäure 12-Hydroxystearinsäure oder eine Mischung aus 12-Hydroxystearinsäure und Ricinolsäure ist.
R4 ist eine Gruppe C(=O)-(R5)-CH(OH)-(CH₂)_{S}-CH₃ oder C(=0)-(R5)- CH[(CH₂)₅-CH₃]-0-{C(=0)-(R5)-CH)-(CH₂)₅- CH₃]-0}ₚC(=O)-(R5)-CH[-(CH₂)₅- CH₃]-OH mit p = 0 bis 5,
R5 ist dabei eine Gruppe (CH₂)₁₀ oder eine Mischung der Gruppen (CH₂)₁₀ und (CH₂)₇- (CH=CH)-CH₂,
mindestens eine der Gruppen RI, R2 und R3 ist dabei eine Gruppe R4.

2. Tensid nach Anspruch 1, ***dadurch gekennzeichnet, dass*** die Verbindung mit der Formel (I) einen durchschnittlichen Polymerisationsgrad n zwischen 4 und 5 hat, vorzugsweise 4,5 bis 5.

3. Tensid nach Anspruch 1 oder 2, ***dadurch gekennzeichnet, dass*** das Tensid einen HLB - Wert zwischen 1 und 6, besser zwischen 2 und 6, und noch besser zwischen 3 und 5 aufweist.

4. Tensid nach einem der Ansprüche**1** bis 3, ***dadurch gekennzeichnet, dass*** p = 0 bis 3, besser p = 0 bis 2.

5. Tensid nach einem der Ansprüche 1 bis 4, ***dadurch gekennzeichnet, dass*** R2 ein Wasserstoffatom ist.

6. Tensid nach einem der Ansprüche 1 bis 5, ***dadurch gekennzeichnet, dass*** es ein Verhältnis zwischen der Zahl der Gruppen R5 = (CH₂)₁₀ und der der Zahl der Gruppen R5 =(CH₂)₇-(CH=CH)-CH₂ zwischen 0,5 und 4, besser zwischen 0,75 und 2 aufweist.

7. Tensid nach einem der Ansprüche 1 bis 6, ***dadurch gekennzeichnet, dass*** RI und R3 R4-Gruppen sind
mit der Formel C(=0)-(CH₂)₁₀ -CH[(CH₂)₅-CH₃]-0-{C(=0)-(CH₂)₁₀-CH[(CH₂)₅-CH₃]-0}_{P}C(=0)-(CH₂)₁₀ -CH[(CH₂)₅-CH₃]-OH
oder
eine Mischung von Gruppen der Formel C(=O)-( CH₂)₁₀-CH[(CH₂)₅-CH₃]-0-{C(=0)-(CH₂)₁₀ -CH[(CH₂)₅-CH_{3]}-0}ₚC(=0)-(CH₂)₁₀-CH[(CH₂)₅-CH₃]-OH und der Formel C(=0)-(CH₂)₇-(CH=CH)-CH₂-CH[(CH₂)₅-CH_{3]}-0-{C(=0)-(CH₂)₇-(CH=CH)-CH₂-CH[(CH₂)₅-CH₃]-0}_{P}C(=0)-(CH₂)₇-(CH=CH)-CH₂-CH[(CH₂)₅-CH_{3]}-OH.

8. Herstellungsverfahren für ein Tensid nach einem der Ansprüche 1 bis 7 durch Verersterungsreaktion zwischen einem Polyglyzerin und mindestens einer Carboxylsäure, bei der Carboxylsäure handelt es sich um 12-Hydroxystearinsäure oder eine Mischung aus 12-Hydroxystearinsäure und Ricinolsäure.

9. Verfahren nach Anspruch 8, ***dadurch gekennzeichnet, dass,*** die Veresterungsreaktion in Anwesenheit eines Molverhältnisses Polyglyzerin / Carboxylsäure zwischen 1 und 0,15, noch besser zwischen 0,5 und 0,25 durchgeführt wird

10. Verfahren nach Anspruch 8 oder 9, ***dadurch gekennzeichnet, dass*** die Veresterungsreaktion bei einer Temperatur zwischen 185 und 235°C, vorzugsweise zwischen 190 und 220°C stattfindet.

11. Tensidzusammensetzung mit dem Tensid nach einem der Ansprüche 1 bis 7.

12. Tensidzusammensetzung mit dem Tensid nach einem der Ansprüche 1 bis 7 oder die Tensidzusammensetzung nach Anspruch 11.

## Claims

1. Water-in-oil surfactant consisting of a mixture of compounds of formula (I): 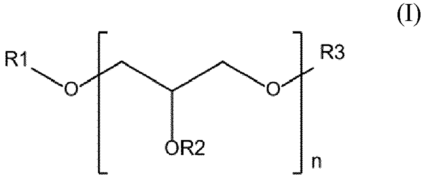
wherein the compound of formula (I) has an average degree of polymerisation n of between 4 and 6,
R1, R2 and R3 being, independently of one another, a hydrogen atom or an R4 group derived from at least one carboxylic acid,
the carboxylic acid being 12-hydroxystearic acid or a mixture of 12-hydroxystearic acid and ricinoleic acid,
R4 being a group C(=O)-(R5)-CH(OH)-(CH₂)₅-CH₃ or C(=O)-(R5)-CH[(CH₂)₅-CH₃]-O-{C(=O)-(R5)-CH[ (CH₂)₅-CH₃]-O}_{P}C(=0)-(R5)-CH[(CH₂)₅-CH₃]-OH where p = 0 to 5,
R5 being a group (CH₂)₁₀ or a mixture of the groups (CH₂)₁₀ and (CH₂)₇-(CH=CH)-CH₂,
at least one of the R1, R2 and R3 groups being an R4 group.

2. Surfactant according to claim 1, ***characterised* in that** the compound of formula (I) has an average degree of polymerisation n of between 4 and 5, advantageously from 4.5 to 5.

3. Surfactant according to claim 1 or 2, ***characterised* in that** the surfactant has a HLB value of between 1 and 6, advantageously of between 2 and 6, more advantageously of between 3 and 5.

4. Surfactant according to one of claims 1 to 3, ***characterised* in that** p = 0 to 3, advantageously p = 0 to 2.

5. Surfactant according to one of claims 1 to 4, ***characterised* in that** R2 is a hydrogen atom.

6. Surfactant according to one of claims 1 to 5, ***characterised* in that** it has a ratio of the number of groups R5 = (CH₂)₁₀ to the number of groups R5 = (CH₂)₇-(CH=CH)-CH₂ of between 0.5 and 4, advantageously between 0.75 and 2.

7. Surfactant according to one of claims 1 to 6, ***characterised* in that** the R1 and R3 groups are R4 groups
of the formula C(=O)-(CH₂)₁₀-CH[(CH₂)₅-CH_{3]}-O-{C(=O)-(CH₂)₁₀-CH[(CH₂)₅-CH₃]-O }ₚC(=O)-(CH₂)₁₀-CH[ (CH₂)₅-CH_{3]}-OH
or
a mixture of groups of the formula C(=O)-(CH₂)₁₀-CH[(CH₂)₅-CH_{3]}-O-{C(=O)-(CH₂)₁₀-CH[(CH₂)₅-CH₃₁-O}ₚC(=O)-(CH₂)₁₀-CH[(CH₂)₅-CH_{3]}-OH and of the formula C(=O)-(CH₂)₇-(CH=CH)-CH₂-CH[(CH₂)₅-CH_{3]}-O-{C(=O)-(CH₂)₇-(CH=CH)-CH₂-CH[(CH₂)₅-CH_{3]}-O}ₚC(=O)-(CH₂)₇-(CH=CH)-CH₂-CH[(CH₂)₅-CH_{3]}-OH.

8. Method for preparing the surfactant according to one of claims 1 to 7 by an esterification reaction between polyglycerol and at least one carboxylic acid, the carboxylic acid being 12-hydroxystearic acid or a mixture of 12-hydroxystearic acid and ricinoleic acid.

9. Method according to claim 8, ***characterised* in that** the esterification reaction is carried out in the presence of a polyglycerol/carboxylic acid(s) mole ratio of between 1 and 0.15, more advantageously between 0.5 and 0.25.

10. Method according to claim 8 or 9, ***characterised* in that** the esterification reaction is carried out at a temperature of between 185 and 235°C, advantageously of between 190 and 220°C.

11. Surfactant composition comprising the surfactant according to one of claims 1 to 7.

12. Cosmetic composition comprising the surfactant according to one of claims 1 to 7 or the surfactant composition according to claim 11.
